(19) Europäisches Patentamt
European Patent Office
Office européen des brevets

(11) Numéro de publication: **0 185 578**
**B1**

# (12) FASCICULE DE BREVET EUROPEEN

(45) Date de publication du fascicule du brevet: **03.10.90**

(51) Int. Cl.$^5$: **C 07 D 207/404**

(21) Numéro de dépôt: **85402298.5**

(22) Date de dépôt: **26.11.85**

## (54) Procédé de synthèse d'esters actifs d'acides carboxyliques, carbonates alpha-halogénés utiles pour cette synthèse et leur mode d'obtention.

(30) Priorité: **04.12.84 FR 8418433**

(43) Date de publication de la demande:
**25.06.86 Bulletin 86/26**

(45) Mention de la délivrance du brevet:
**03.10.90 Bulletin 90/40**

(84) Etats contractants désignés:
**CH DE FR GB IT LI NL SE**

(56) Documents cités:
**EP-A-0 002 677**
**EP-A-0 082 404**
**EP-A-0 010 297**
**FR-A-2 358 394**

**CHEMISCHE BERICHTE, vol. 100, 1967, pages 16-22, Verlag Chemie GmbH, Weinheim, DE; E. VOWINKEL: "Darstellung von Carbonsäureestern mittels O-Alkyl-N.N'-dicyclohexyl-isoharnstoffen"**

**TETRAHEDRON, vol. 36, no. 17, 1980, Pergamon Press, Ltd., Oxford, US; E. HASLAM: "Recent developments in methods for the esterification and protection of the carboxyl group"**

(73) Titulaire: **SOCIETE NATIONALE DES POUDRES ET EXPLOSIFS**
**12, quai Henri IV**
**F-75181 Paris Cédex 04 (FR)**

(72) Inventeur: **Barcelo, Gérard**
**3 rue Mozart Résidence Claire-Joie**
**F-91700 Sainte Genevieve des Bois (FR)**
Inventeur: **Castro, Bertrand**
**L'Estaple Avenue des Costières**
**F-34130 Saint Aunes (FR)**
Inventeur: **Jaouadi, Mahmoud**
**Chez Monsieur Amara Zouaoui El Amra**
**G. de Sfax (TN)**
Inventeur: **Martinez, Jean**
**Rue des Cévennes**
**F-34720 Caux (FR)**
Inventeur: **Senet, Jean-Pierre**
**79, rue de la Gare Herbeauvilliers-Buthiers**
**F-77760 La Chapelle La Reine (FR)**
Inventeur: **Sennyey, Gérard**
**1, place des Quatre Pavés**
**Saint-Aubin F-91190 Gif Sur Yvette (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 185 578 B1**

Courier Press, Leamington Spa, England.

**Description**

L'invention concerne une nouvelle synthèse d'esters actifs d'acides carboxyliques. Elle concerne également les nouveaux carbonates alphahalogénés utiles pour cette synthèse et leur mode d'obtention.

Les esters actifs d'acides carboxyliques sont très recherchés en particulier dans le domaine de la synthèse peptidique. Ainsi les acides aminés N-protégés estérifiés sont des produits stables qui pourront être stockés et commercialisés. Il suffira ensuite de les faire réagir sur un composé aminé pour forme la liaison peptidique.

Les différents type d'esters actifs sont bien décrits dans la littérature (Houben Weyl Vol 15 2ème partie ou Specialist Periodical Reports "Amino-acides Peptides and Protein" Vol 1—14).

Parmi les plus courants on peut citer les esters aryliques substitués tels que les esters de 2,4,5-trichlorophényle et les esters d'hydroxyamines tels que les esters de N-succinimidyle.

Ces esters actifs peuvent également être utilisés pour le couplage de molécule biologiquement actives sur les protéines ou des supports solides ou liquides (demande de brevet allemand n° 2631656, demande de brevet européen n° 2677).

Ils le sont encore pour le greffage d'acides aminés sur des molécules biologiquement actives (demande de brevet européen n° 10297).

Un certain nombre de procédés ont déjà été proposés pour les préparer (Tetrahedron *36* 2409 (1980)).

Le plus utilisé consiste à condenser l'acide et le composé hydroxylé en présence de dicyclohexylcarbodiimide comme agent déshydratant mais les rendements sont variables et il se forme, comme produit secondaire, de la dicyclohexylurée qui et trés difficile à éliminer complètement et des N-acylurées (Chem. Ber. 100, 16 (1967)).

Selon un autre procédé on fait réagir le composé hydroxylé sur l'anhydride mixte formé à partir de l'acide à estérifier et d'un chlorure d'acide carboxylique, carbonique ou phosphorique. Deux étapes sont alors généralement nécessaires. Les chlorures d'acides sont des réactifs peu agréables à manipuler et souvent toxiques.

Il est aussi possible d'obtenir les esters actifs par réaction de l'acide sur un carbonate symétrique formé à partir du composé hydroxylè. Mais on perd alors un équivalent du composé hydroxylè, souvent un produit cher, qui est difficile à éliminer du milieu et à récupérer.

Il a aussi été proposé de remplacer le composé hydroxylé, dans le cas du 2—4 dinitrophénol par son fluorure qui réagit sur le sel de sodium de l'acide. Mais cette méthode est très spécifique.

Certains auteurs ont quant à eux mis en oeuvre des réactifs plus sophistiqués tels que les sels de 2-halopyridinium ou le couple triphénylphosphine/diéthyle azodicarboxylate.

Le très grand nombare de procédés proposés dans la littérature pour l'obtention de ces esters actifs tend à prouver qu'aucun n'est vraiment complètement satisfaisant et que vu l'intérêt de ces composés on est toujours à la recherche d'une solution meilleure.

L'objet de l'invention est précisément de proposer un procédé qui répond à cette attente.

Selon le procédé de l'invention on fait réagir sur un acide carboxylique de formule Y—COOH dans laquelle Y est le reste d'un amino-acide N-protégé, d'un acide aliphatique en $C_1$ à $C_6$, saturé ou non, d'un acide hétérocyclique à 5 ou 6 chaînons, saturé ou non, les hétérotomes étant choisis parmi le soufre ou l'azote, d'un acide aromatique hydrocarboné à 6 chaînons, en présence d'un agent de fixation d'acide chlorhydrique, un carbonate de formule

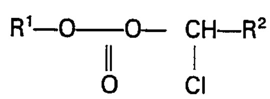

dans laquelle $R^1$ représente

un radical de formule

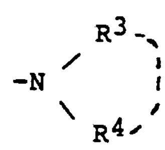

dans laquelle $R^3$ et $R^4$ identiques ou différents sont des radicaux hydrocarbonés pouvant comporter des atomes de carbone doublement liés à un atome d'oxygène et sont reliés entre eux pour former avec l'atome d'azote un hétérocycle azoté ayant jusqu'à 5 atomes de carbone dans le cycle, saturé ou non, qui peut être condensé avec un cycle phényle,

un radical benzotriazole,

et $R^2$ représente un radical aliphatique en $C_1$ à $C_5$ substitué ou non par un ou plusieurs atomes d'halogène ou le radical phényle substitué par un ou plusieurs atomes de chlore,

ou dans laquelle $R_1$ représente

un radical phényle substitué ou non par des atomes d'halogène et/ou des groupes nitro;

et $R^2$ représente un radical — $CCl_3$ ou un radical phényle substitué par un ou plusieurs atomes de chlore.

Le schéma réactionnel es le suivant:

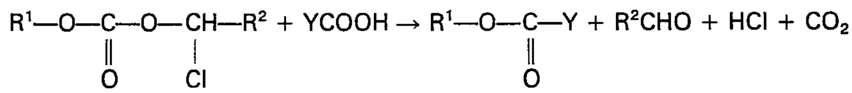

Cette réaction est surprenante car en effet il est connu que des carbonates alpha-halogénés réagissent sur des acides pour donner des ester carbonates suivant le schéma:

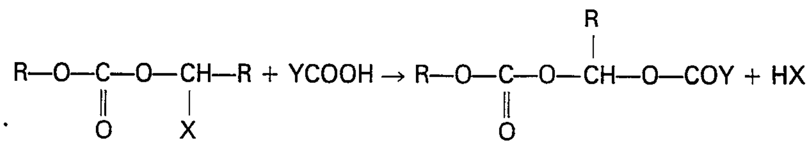

Cette réaction est décrite notamment dans les brevets FR 2 201 870 et EP 82404 et est très utile pour améliorer l'efficacité de certains médicaments.

Le mécanisme de la réaction selon l'invention et totalement différent et c'est un mérite de l'invention d'avoir découvert les conditions qui permettent d'obtenir des esters actifs.

Le procédé selon l'invention s'applique à un très grand nombre d'acides simples ou complexes, acides aliphatiques, cycloaliphatiques, hétérocycliques, saturés ou non, aussi bien qu'aromatiques. On peut à titre d'exemple citer l'acide acétique, acrylique, thiophène carboxylique, benzoïque.

Il est particulièrement intéressant pour l'estérification des acides aminés naturels ou synthétiques N-protégés.

Si les acides portent des fonctions particulièrement réactives il est préférable de les protéger mais ce n'est généralement pas nécessaire.

Les carbonates alpha-halogénés de formule

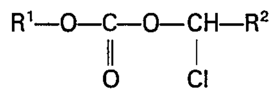

dans laquelle $R^1$ et $R^2$ ont les significations précédentes, que l'on fait réagir sur les acides sont des composés nouveaux qui font également l'objet de la présente invention.

Lorsque $R^1$ représente un radical phényle substitué par des atomes d'halogène, ce sont en particulier des atomes de chlore ou de fluor.

Les radicaux qui sont considérés comme des bons groupes activants dans les esters actifs sont les radicaux préférés. Ce sont par exemple le N-succinimidyle, le paranitrophényle, le 2,4-dinitrophényle, le 2,4,5-trichlorophényle, le pentachloro- ou fluorophényle, le N-phtalimidyle.

Lorsque $R^2$ représente un radical aliphatique, il représente en particulier le radical méthyle, qui peut être substitué par un ou plusieurs atomes d'halogène. Le radical trichlorométhyle convient bien.

Les carbonates alpha-halogénés de l'invention sont généralement cristallins, stables à la température ambiante, peu ou pas toxiques.

Leur mode d'obtention qui fait également l'objet de l'invention sera décrit un peu plus loin.

Une de leur application est la préparation d'esters actifs come précédemment décrit.

On utilise généralement dans cette application un équivalent de carbonate pour un équivalent d'acide. On peut s'écarter si besoin est de cette proportion.

Le carbonate et l'acide sont mis à réagir dans un solvant inerte à leur égard. Ce solvant est généralement choisi parmi les éthers cycliques ou non, comme le tétrahydrofuranne ou le dioxanne, les esters comme par exemple l'acétate d'éthyle, les nitriles comme l'acétonitrile, les alcools comme par exemple l'isopropanol les amides comme par exemple la diméthylformamide et les cétones comme par exemple l'acétone. Le tétrahydfuranne convient bien.

La présence d'un agent de fixation d'acide halohydrique est nécessaire pour éliminer l'acide qui se forme au cours de la réaction. Tous les moyens habituels peuvent être employés. Ce sera généralement une base minérale, par exemple le carbonate de potassium ou organique par exemple une amine tertiaire.

Parmi les bases préferées on peut citer la N-méthyl morpholine ou la triéthylamine.

On utilise généralement un équivalent de base par équivalent d'acide mais un excès de base n'est pas un inconvénient.

La température de réaction est généralement comprise entre −20° et 100°C. Elle est choisie en fonction de la réactivité du carbonate utilisé. Le plus souvent elle est comprise entre 20° et 30°C.

Le temps de réaction quant à lui varie de quelques minutes à quelques heures. Généralement deux heures suffisent pour obtenir de bons résultats. Un moyen simple pour constater la fin de la réaction est d'observer l'interruption du dégagement de gaz carbonique.

L'acide estérifié obtenu est purifié par des méthodes classiques et simples. Les sous-produits de la

réaction qui sont le chlorhydrate de la base utilisée et l'aldéhyde sont facilement éliminés, par exemple par lavage à l'eau. Ensuite par évaporation du solvant on obtient déjà un produit d'une grande pureté qui peut être recristallisé dans un solvant approprié. Les rendements sont excellents.

L'invention concerne également le mode d'obtention des nouveaux carbonates alpha-halogénés précédemment décrits.

Ce mode d'obtention consiste à faire réagir le composé hydroxylé de formule $R^1OH$ dans laquelle $R^1$ a la signification précédente avec un chloroformiate alpha-halogéné de formule

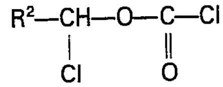

dans laquelle $R^2$ a la signification précédente, dans un milieu solvant inerte, à une température comprise entre −20° à 100°C en présence d'une base organique ou minérale.

La réaction est la suivante:

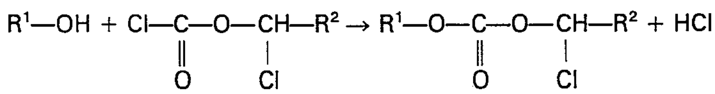

Les composés hydroxylés et les composés alpha-halogénés de départ se trouvent en générale dans le commerce ou se fabriquent selon des méthodes connues. Par exemple les chloroformiates alpha-halogénés sont préparés par halogénation des chloroformiates correspondants ou comme décrit dans les demandes de brevet français n° 2 482 587, 2 532 933, européen 108 547.

Comme base organique ou minérale on emploie une base habituellement connue pour fixer l'acide chlorhydrique. De préférence on utilise la pyridine ou la triéthylamine.

Il est préférable d'ajouter la base au mélange des réactifs et également de l'ajouter progressivement.

On utilise généralement une quantité stoechiométrique de la base et des composants réactifs mais on peut employer un excès d'alcool.

Le milieu solvant est constitué d'un ou de plusieurs solvants inertes vis-à-vis des réactifs. On choisit de préférence les solvants aliphatiques chlorés par exemple le dichlorométhane, le dichloroéthane, les éthers cycliques ou non, les cétones telles que l'acétone ou la butanone-2, les nitriles, les esters et les hydrocarbures aliphatiques ou aromatiques.

Lorsqu'on fait réagir certains composés hydroxylés tels que les phénols il est préférable de choisir un couple de solvant dans lequel le phénol soit peu soluble et le chlorhydrate de la base insoluble. Le mélange benzène-éther de pétrole par exemple convient bien.

La température de la réaction est de préférence comprise entre −10° et 40°C.

A la fin de l'introduction des réactifs on porte généralement le mélange à la température ambiante. Le temps de réaction est souvent compris entre quelques minutes et quelques heures.

Le carbonate obtenu et facilement isolé par les méthodes usuelles. Par exemple le mélange est lavé à l'eau glacée ou au sulfate acide de sodium, ou filtré sur un lit de celite puis le ou les solvants sont évaporés. Les cristaux généralement obtenus peuvent être recristallisés de façon classique.

Ainsi l'invention pemet de préparer de nouveaux carbonates de façon simple sans installation particulière. Ces nouveaux carbonates grâce à leur structure originale pourront faire l'objet d'applications très variées.

L'application à l'estérification des acides qui fait également l'objet de la présente invention est une application très intéressante. Les esters acifs des acides sont obtenues ainsi sans grande difficulté et sans racémisation en utilisant de façon simple des réactifs faciles à obtenir et à manipuler. Les sous-produits de la réaction, chlorhydrate de la base utilisée et aldéhyde sont aisément éliminés. Les rendements sont très bons et les produits d'une grande pureté.

Les exemples qui suivent sont donnés à titre illustratif.

*Exemples 1 à 10;* Synthèse des carbonates alpha-halogénés.

On prépare les carbonates selon une ou plusieurs des méthodes suivantes. Les résultats sont rassemblés dans le tableau 1.

Methode A

On ajoute en une seule fois 12,35 g (0,05 mol) de chloroformiate de 1,2,2,2-tétrachloréthyle à une solution de 5,75 g (0,05 mol) de N-hydroxysuccinimide dans le dichlorométhane (50 ml). On refroidit à 0°C et on ajoute goutte à goutte 4 g (0,05 mol) de pyridine. A la fin de l'addition on laisse revenir à température ambiante et on agite pendant 3 heures. On ajoute alors 20 ml d'eau glacée, on sépare la phase organique qu'on lave avec autant de fois 20 ml d'eau glacée qu'il est nécessaire pour que le pH ne soit plus acide (en général 3 à 4 fois). On sèche la phase organique sur sulfate de magnésium, on évapore et on obtient un solide blanc qu'on cristallise dans l'éther de pétrole. On recueille 13,5 g (rendement R = 83%) du carbonate attendu. On peut omettre cette recristallisation; le rendement est alors de 94% et le produit est d'une pureté satisfaisante.

Methode B

On opère comme dans la méthode A en remplacant la pyridine par la triéthylamine. On fait réagir une heure à 0°C puis une heure à température ambiante. La phase organique est ici lavée rapidement avec une solution saturée de $NaHSO_4$. Après évaporation, le carbonate de N-hydroxysuccinimide es cristallisé dans l'éther éthylique (R = 83%).

Methode C

On ajoute 27 g (0,11 mol) de chloroformiate de 1,2,2,2 tétrachloroéthyle à une suspension de 18,4 g (0,1 mol) de 2,4 dinitrophénol dans 150 ml de benzène et 150 ml d'éther de pétrole. On refroidit à 0°C et on ajoute 11 g (0,11 mol) de triéthylamine goutte à goutte et en maintenant une agitation violente. On agite 4 heures à 20°C puis on filtre sur un lit de celite. On évapore les solvants et on obtient 26 g (R = 66%) de cristaux blancs après lavage avec un peu d'éther de pétrole.

TABLEAU 1

| Ex | Produit | Méthode | Rendement | Solvant de cris-talli-sation | F ou EmmHg °C | RMN 1H ppm | IR $cm^{-1}$ | Analyse élémentaire |
|---|---|---|---|---|---|---|---|---|
| 1 | 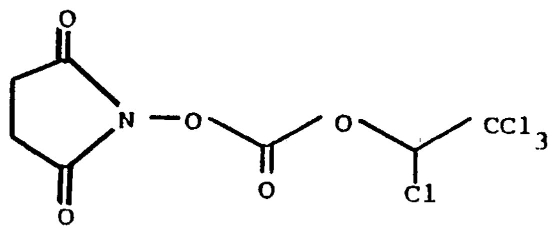 | A<br>B | 83<br>83 | Ether de pétrole<br>Ether éthy-lique | 104<br>108 | 2,9 (s)<br>6,6 (s) | 1760 | C 26,00; H 1,74; N 4,10; O 23,54; Cl 43,55; (calculée: C 25,87; H 1,55; N 4,31; O 24,6; Cl 43,55; |
| 2 | 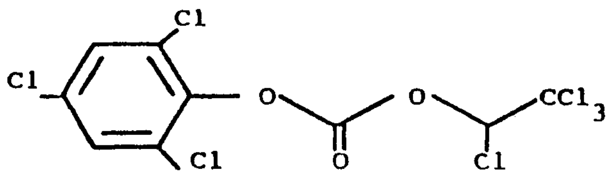 | A | 64 | Ether de pétrole | 71 | 6,73 (s)<br>7,4 (s) | 1800 | C 26,61; H 1,02; O 11,56; Cl 60, 80; (calculée: C 26,54; H 0,74; O 11,78; Cl 60,93) |
| 3 | 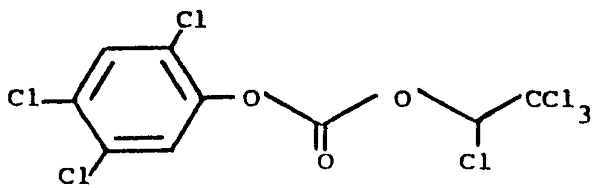 | A | 92% brut<br>70% dist. | — | E0,02 = 150 – 155 | 6,7 (s)<br>7,4 (s)<br>7,56 (s) | 1795 | C 26,67; H 0,90; O 11,95; Cl 60,64; (calculée: C 26,54; H 0,74; O 11,78; Cl 60, 93) |
| 4 | 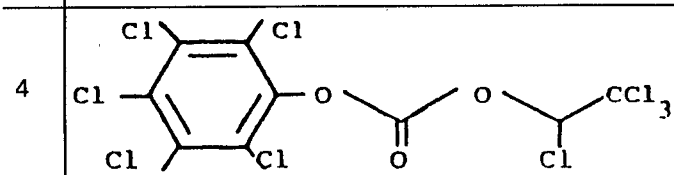 | A | 98 | AcOEt | 120 | 6,7 (s) | 1800 | C 22,40; H trace; Cl 67,15; (calculée: C 22,66; H 0,27; Cl 67,05) |
| 5 | 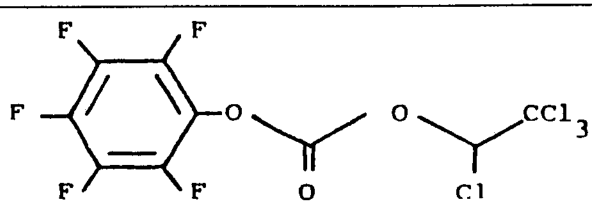 | A | 91 | — | E0,05 = 80 | 6,7 (s) | 1800 | C 27,60; H 0,47; Cl 35,80; F 24,56; (calculée C 27,44; H 0,26; Cl 36,00; F 24,12) |

TABLEAU I (Suite)

| Ex | Produit | Méthode | Rendement | Solvant de cristallisation | F ou EmmHg °C | RMN 1H ppm | IR $cm^{-1}$ | Analyse élémentaire |
|---|---|---|---|---|---|---|---|---|
| 6 | 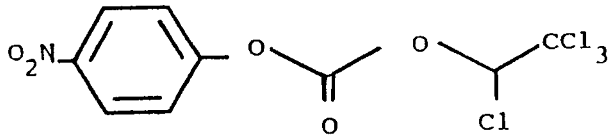 | C | 75 | Ether de pétrole | E 0,05 = 165<br>F = 69 | 6,7 (s)<br>7,5 (d)<br>8,36 (d) | 1790 | C 30,87; H 1,53; N 3,81; O 22,23; Cl 41, 15; (calculée: C 30,97; H 1,44; N 4,01; O 22,92; Cl 40,64) |
| 7 | 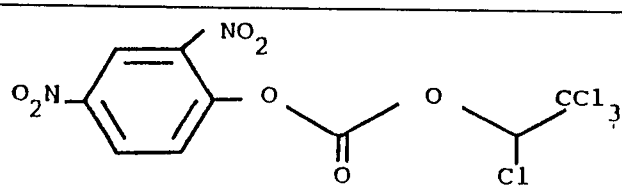 | C | 66 | Ether de pétrole | 121—122 | 6,73 (s)<br>8,60 (s)<br>9,06 (d)<br>7,66 (d) | 1795 | C 27,49; H 1,04; N 6,98; O 27.99; Cl 35,95; (calculée: C 27,44; H 1,02; N 7; O 28,43; Cl 36,00) |
| 8 | 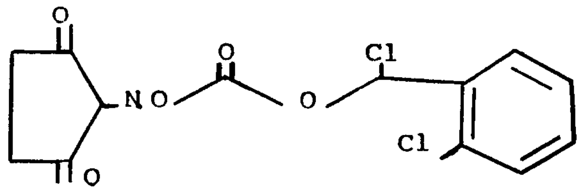 | A | 75 | | E 0,05 = 115—131 | 2,8 (s)<br>7,2 (s)<br>7,3 à 8 (m) | 1750 | $M^+$ + 18 = 335 |
| 9 | 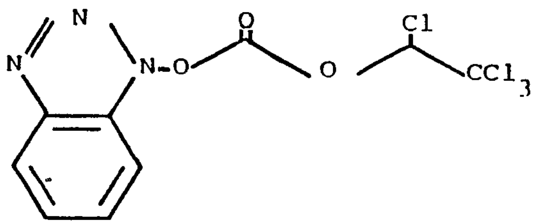 | B | 85 | $CH_2Cl_2$ | 145—147 | 7,04 (s)<br>7,6 (dd)<br>7,8 (dd)<br>8,0 (d)<br>8,2 (d) | 1770<br>1790 | C 31,38; H 1,37; N 12,16; Cl 41,03; (calculée C 31,30; H 1,45; N 12,17; Cl 41,16) |
| 10 | 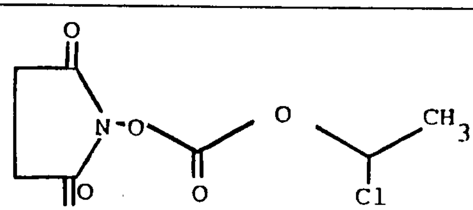 | A | 86 | Ether de pétrole | 105—106 | 6,4 (q)<br>1,9 (d)<br>2,8 (s) | 1750 | C 37,80; H 3,64; N 6,24; O 36,68; Cl 15,88; (calculée C 37,94; H 3,64) N 6,32; O 36,10; Cl 16,00 |

Exemple 11

Préparation du N-(tertio butyloxycarbonyl)-L-alaninate de N-succinimidyle.

On dissout à 0,95 (5 mmol) de N-(tertio butyloxycarbonyl)-L-alanine et 0,66 ml (5,5 mmol) de N-méthylmorpholine dans 6 ml de THF et on ajoute d'un coup 1,8 g (5,5 mmol) de carbonate de N-succinimidyle et de 1,2,2,2-tétrachloroéthyle. On agite pendant deux heures à 20°C. On ajoute environ 25 ml d'acétate d'éthyle et on lave rapidement la phase organique avec une solution d'HCL N, puis avec une solution de bicarbonate de potasium et enfin deux fois à l'eau. On sèche sur sulfate de magnésium et on évapore. On cristallise le résidu dans un mélange acétate d'éthyle-éther de pétrole et on obtient 1,25 g (R = 87%) de cristaux blancs. L'évaporation des eaux mères et la cristallisation permet de récupérer encore 0,1 g ce qui porte le rendement à 94%.

F (point de fusion) = 158°C $[\alpha]_D^{20} = -50,7$ (c = 2, dioxane)

Exemple 12

Préparation du N α-(tertiobutyloxycarbonyl)-Nε-(benzyloxycarbonyl)-L-lysinate de N-succinimidyle.

On ajoute 1,64 g (5 mmol) de carbonate de N-succinimidyle et de 1,2,2,2-tétrachloroéthyle à une solution de 1,9 g (5 mmol) de Nα-BOC − Nε − Z − lysine et de 0,7 ml (5 mmol) de triéthylamine dans 15 ml de THF. On agite à température ambiante pendant 2 heures. On rajoute alors 20 ml d'acétate d'éthyle et on lave la phase organique avec une solution d'acide citrique 0,5 N, puis trois fois avec une solution à 5% de bicarbonate de sodium et enfin avec une solution saturée de chlorure de sodium. On sèche sur sulfate de sodium et on évapore à sec. On obtient 2,03 g (R = 85%) de cristaux blancs.

F = 110°C $[\alpha]_D^{20} = -21,51$ (c = 2, dioxane)

| | |
|---|---|
| Analyse calculée pour $C_{23}H_3N_3O_8O, 5H_2O$: | C = 56,79; H = 6,79; N = 8.64 |
| Trouvée: | C = 56,32; H = 6,44; N = 8,77 |

Exemples 13 à 29

On prépare comme à l'exemple 11 ou 12 d'autres esters de N-succinimidyle. Tous les résultats sont résumés dans le tableau 2.

TABLEAU 2 Esters de N-succinimidyle

| EX | Acide aminé | Rendement % | F°C | $[\alpha]_D^{20}$ (c, solvant) | Rendement littérature % | F°C | $[\alpha]_D^{20}$ (c, solvant) | REF |
|---|---|---|---|---|---|---|---|---|
| 11 | BOC-Ala | 94 | 158 | −50,7 (2, dioxane) | 71 | 161 | −49,0 (2, dioxane) | 1 |
| 12 | BOC-(Z)-Lys | 85 | 110 | −21,51 (2, dioxane) | — | 110 | −21,12 (2, dioxane) | 2 |
| 13 | BOC-Phe | 91 | 147 | −19,8 (2, dioxane) | 81 | 152 | −20,7 (2,2, dioxane) | 1 |
| 14 | BOC-Gly | 91 | 162 | — | 62 | 168 | — | 1 |
| 15 | BOC-Val | 92 | 125 | −37,4 (2, dioxane) | 74 | 125 | -37 (2, dioxane) | 1 |
| 16 | BOC-Pro | 82 | 133 | −54,7 (2, dioxane) | 74 | 135 | −55,3 (2, dioxane) | 1 |
| 17 | BOC-Tyr | 69—74 | 181 | −12,12 (1, dioxane) | | 180 | −12,5 (1, dioxane) | 2 |
| 18 | BOC-Trp | 89 | 140 | −23,13 (2, dioxane) | 37 | 153 | 22,4 (2, dioxane) | 1 |
| 19 | BOC-Met | 92 | 124 | −21,6 (2, dioxane) | 59 | 128 | −20,6 (2, dioxane) | 1 |
| 20 | BOC-(SBzl) Cys | 61 | | −54,0 (2, dioxane) | 49 | 117 | −54,0 (2, dioxane) | 1 |
| 21 | BOC-(OBzl)-Ser | 78 | — | + 5,8 (0,5, dioxane) | 52 | 112—113 | $-[\alpha]_D^{26}$= + 6,5 (0,5, dioxane) | 2 |
| 22 | BOC-(OBzl)-Thr | 85 | — | + 9.38 (2, dioxane) | — | 94 | −8,5 (1, méthanol) | 2 |
| 23 | BOC-(OBzl) Tyr | 73 | 144 | − 6,2 (2, dioxane) | — | 149 | −6,3 (2, dioxane) | 2 |
| 24 | Z-Ala | 86 | 123 | −36,9 (2, dioxane) | 65 | 123 | −37,2 (2, dioxane) | 1 |
| 25 | Z-Pro | 97 | 90 | −54,9 (2, dioxane) | 74 | 90 | −54 (2, dioxane) | 1 |
| 26 | Z-Met | 72 | 102 | −16,2 (2, dioxane) | 59 | 102 | −15,9 (2, dioxane) | 1 |
| 27 | FMOC-Ala | 70 | 102 | — | — | — | — | — |
| 28 | FMOC-Phe | 97 | 137 | — | — | — | — | — |
| 29 | FMOC-Pro | 100 | | — | — | — | — | — |

Ref. 1) J. Am. Chem. Soc 86, 1839 (1964)
2) Houben-Weyl: Methoden der Org. Chemie Vol. 15(2) p. 165 et loc. Citées

Exemple 30

Préparation du N-(tertiobutyloxycarbonyl)-L-phénylalaninate de N-succinimidyle

A une solution de 1,33 g (5 mmol) de BOC-L-phénylalanine dans 6 ml de THF, on ajoute 0,8 g de carbonate de potasium anhydre et 1,8 g de carbonate de 1,2,2,2 tétrachloroéthyle et de N-succinimidyle. On agite deux heures à 20°C et on traite comme à l'exemple 11. On obtient 1,15 g (R = 63%) de BOC-L-PheOSu. F = 135°C.

Si on utilise 1 ml d'une solution aqueuse saturée de carbonate de potassium, à la place du carbonate de potassium anhydre, on obtient 1,3 g (R = 72%) de l'ester précédent.

Exemple 31

Préparation du N-tertiobutyloxycarbonylglycinate de N-succinimidyle.

On opère comme à l'exemple 11, en remplacant le THF par l'acétonitrile. A partir de 1,76 g de BOC-glycine, on obtient 2,1 g (R = 77%) de BOC-Gly-OSu.

F = 158°C F litt = 168°C

Exemple 32

Préparation du N-tertiobutyloxycarbonyl)-L-phénylalaninate de N-succinimidyle

A une solution de 1,33 g (5 mmol) de BOC-L-phénylalanine dans 6 ml de THF, on ajoute 0,66 ml de N-méthylmorpholine et 1,11 g (5 mmol) de carbonate de 1-chloroéthyle et de N-succinimidyle. On agite 16 heures à 20°C et on traite comme à l'exemple 11. On obtient 1,40 g (R = 77%) de BOC-L-Phe-OSu.

F = 143°C $[\alpha]_D^{20} = -16{,}6$ (c = 2,2 dioxane)

Exemple 33

Préparation du N-tertiobutyloxycarbonyl)-L-alaninate de 2-chlorophényl chlorométhyle

A une solution de 0,95 g (5 mmol) de BOC-L-alanine et 0,66 ml de N-méthyl morpholine dans 6 ml de THF, on ajoute 2,0 g de carbonate de 2-chlorophényle chlorométhyle et de N-succinimidyle. On agite 1 heure 30 minutes à 20°C et on traite comme à l'exemple 11. On obtient 1,25 g (R = 90%) de BOC-L-Ala-OSu.

F = 158—159°C $[\alpha]_D^{20} = -49{,}5$ (c = 2 dioxane)

Exemples 34 à 42

Préparation des esters de 2,4,5-trichlorophényle

Ces esters sont préparés comme à l'exemple 11 ou 12, à partir du carbonate de 1,2,2,2-tétrachloroéthyle et de 2,4,5-trichlorophényle. Les résultats sont rassemblés dans le tableau 3.

Exemple 43

Préparation du N-tertiobutyloxycarbonyl)-L-alaninate de pentachlorophényle

On procède comme à l'exemple 12. A partir de 0,84 g (5 mmol) de BOC-L-alanine et de 2,27 g (5 mmol) de carbonate de pentachlorophényle et de 1,2,2,2-tétrachloroéthyle, on obtient 2,06 g (R = 94%) de cristaux blancs (solvant de recristallisation: acétate d'éthyle-hexane).

F = 170°C $[\alpha]_D^{20} = -24{,}47$ (c = 1, $CHCl_3$)

littérature

F = 166°C $[\alpha]_D^{20} = -22{,}2$ (c = 5,1, $CHCl_3$)

Exemple 44

Préparation du N-tertiobutyloxycarbonyl-Nε-benzyloxycarbonyl-L-lysinate de pentachlorophényle

On procède come à l'exemple 12. A partir de 1,9 g de Nα-BOC-Nε-Z-L-lysine, on obtient 2,4 g (R = 77%) de Nα-BOC-Nε-Z-L-lysine-OPCP.

TABLEAU 3
Esters de 2,4,5-Trichlorophenyle

| EX | Acide aminé | Rendement % | F°C | $[\alpha]_D^{20}$ (c, solvant) | Rendement littérature | F°C | $[\alpha]_D^{20}$ (c, solvant) littérature % | REF |
|---|---|---|---|---|---|---|---|---|
| 34 | BOC-Ala | 79 | 82 | −43,5 (2, ac. acétique) | 73 | 82 | −44 (2, ac. acétique) | 1 |
| 35 | BOC (SBzl) Cys | 60 | — | | 86 | 77 | — | 1 |
| 36 | BNOC (DNP) Hist. | 58 | 98 | + 1,87 (2, dioxane) | — | — | — | |
| 37 | BOC-(OBzl) Glu | 60 | 107 | −33,5 (2, méthanol) | 60 | 108 | −33,5 (2, méthanol) | 2 |
| 38 | BOC-(Z) Lys | 85 | 99 | −19,13 (2, dioxane) | 86 | 99 | — | 1 |
| 39 | BOC-Met | 79 | 90 | −38.0 (2, DMF) | 71 | 91 | −38,5 (2, DMF) | 1 |
| 40 | BOC-Ser | 22 | 105 | −42,0 (2, DMF) | 5 | 105 | −41 (2, DMF) | 3 |
| 41 | BOC-(OBz-1) Ser | 77 | Huile | + 0,3 (2, dioxane) | — | — | — | — |
| 42 | BOC-Tyr. | 73 | 168 | 31,4 (3,5, ac. acétique) | 40 | 158 | −26,4 (3,5 ac. acétique) | 2 |

Ref. 1) J. Chem. Soc (C) 1967) — 2) Tetrahedron Suppl. 8,39 (1966) — 3) J. Med. Chem. 10, 1047 (1977)

$F = 142°C\ [\alpha]_D^{20} = -16,7\ (c = 1,\ CHCl_3)$

littérature:
$F = 141°C\ [\alpha]_D^{20} = -15,0\ (c = 4,99,\ CHCl_3)$

Exemple 45

Préparation du n-tertiobutyloxycarbonyl-L-phenylalaninate de 4-nitrophényle

On dissout 0,73 g (2,75 mmol) de BOC-L-phénylalanine et 0,3 ml de N-methyl morpholine dans 6 ml de THF et on ajoute à 87 g (2,5 mmol) de carbonate de 1,2,2,2-tétrachloroéthyle et de 4-nitrophényle. On agite une heure à température ambiante. On ajoute alors 15 ml d'acétate d'éthyle et on lave avec une solution d'acide chlorhydrique normal puis avec une solution saturée de chlorure de sodium. On sèche sur sulfate de magnésium. On évapore à sec et on cristallise dans l'éthanol à 95%. On obtient 0,6 g (R = 62%) de cristaux blancs.

$F = 118°C\ [\alpha]_D^{20} = -20,9\ (c = 2,\ DMF)$

littérature:
$F = 132°C\ [\alpha]_D^{20} = -21\ (c = 2,\ DMF)$

Exemple 46

Préparation du n-tertiobutyloxycarbonyl-L-alaninate de 2,4 dinitrophényle

On procède comme dans l'exemple précédent. A partir de 0,945 g (5 mmol) de N-BOC-L-alanine, on obtient 1,45 g (R = 82%) de N-BOC-L-Ala-O-2,4 DNP cristallisé dans l'alcool éthylique à 95%.

$F = 95—96°C\ [\alpha]_D^{20} = -51,5\ (c = 0,2,\ DMF)$

Exemple 47 à 54

Prèparations des esters de pentafluorophényle

On procède comme à l'exemple 11. Les résultats sont rassemblés dans le tableau 4.

TABLEAU 4
Esters de Pentafluorophenyle

| EX | Amino-acides | Rendement % | F°C | Litté-rature F°C | $[\alpha]_D^{20}$ (c, solvant) | Littérature $[\alpha]_D^{20}$ (c, solvant) | REF. |
|---|---|---|---|---|---|---|---|
| 47 | BOC-Ala | 90 | 85 | 83 | −34,6 (1, dioxane) | −31,2 (1, dioxane) | 1 |
| 48 | BOC-Gly | 90 | 80 | 80 | — | — | |
| 49 | BOC-Phe | 86 | 112 | 112 | −16,9 (1, dioxane) | −26,9 (1, dioxane) | 1 |
| 50 | BOC-Val | 94 | 64 | 64 | −31,8 (1, dioxane) | −18,4 (1, dioxane) | 1 |
| 51 | BOC-Pro | 98 | 50 | 51 | −48,2 (1, dioxane) | −53 (1, dioxane) | 1 |
| 52 | BOC-Trp | 91 | 111 | 111 | −12,8 (1, dioxane) | −28,1 (1, dioxane) | 1 |
| 53 | BOC-Met | 81 | 79 | 80 | −20,7 (1, dioxane) | −22,5 (1, dioxane) | 1 |
| 54 | Z-Pro | 100 | huile | huile | −55,08 (1, dioxane) | −55,05 (1, dioxane) | 1 |

Ref. 1) Ann. 1421 (1973)

Exemple 55

Préparation du N-(tertiobutyloxycarbonyl)-L-phénylalaninate de pentafluorophényle.

On opère comme à l'exemple 48 mais en variant le solvant de réaction. Les résultats obtenus sont rassemblés dans le tableau suivant:

| Solvant | Rendement | F°C | $[\alpha]_D^{20}$ |
|---|---|---|---|
| THF | 86 | 112 | −16,9 |
| Dioxane | 86 | 113 | −16,4 |
| Acetate d'ethyle | 83 | 112 | −15,4 |
| DMF | 65 | 107 | −14,2 |

Exemples 56 et 57

Ces exemples illustrent l'utilisation des esters actifs des acides aminés à la synthèse des di-et tripeptides.

56. Préparation du N-(BOC-L-phénylalanyglycinate d'éthyle (BOC-Phe-Gly-OEt).

A une solution de 0,86 g (2 mmol) de N-(t-butoxycarbonyl-L-phénylalaniate de pentafluorophényle, préparé selon l'exemple 48, dans 10 ml de dioxane, on ajoute simultanément 0,28 g (2,2 mmol) de glycinate d'éthyle (chlorhydrate) et 0,31 g (3 mmol) de triéthylamine. On agite une heure à température ambiante. On dilue avec 10 ml d'acétate d'éthyle et on lave avec des solutions aqueuses d'acide chlorhydrique 0,1 N, de bicarbonate de potasium, et de chlorure de sodium. On sèche sur sulfate de magnésium et on évapore. On reprend par un mélange 4/6 d'acétate d'éthyle-hexane et on filtre sur silice. Après évaporation, on obtient 0,53 g (R = 76%) de BOC-Phe-Gly-OEt.

$$F = 89°C\ [\alpha]_D^{20} = -4{,}8\ (c = 5{,}0\ EtOH)$$

$$F_{litt} = 89{,}5°C\ [\alpha]_D^{20} = -4{,}2\ (c = 5{,}0\ EtOH)$$

(J. Amer. Chem. Soc. *82,* 4596 (1960)

57. Préparation du N-benzyloxycarbonyl-prolinyl-glycyl-glycinate d'éthyle.

Z-pro-GlyOH → Z-Pro-Glyo-OPFP → Z-Pro-Gly-Gly-OEt

On ajoute 1,4 g (3,6 mmol) de carbonate de 1,2,2,2 tétrachloroéthyle et pentafluorophenyle à une solution de 1,07 g (3,6 mmol) de N-benzyloxycarbonyl-prolinyl-glycine et 0,4 ml de N-méthylmorpholine dans 5 ml de dioxane. On agite une heure à 20°C et on traite comme à l'exemple 55. On obtient 0,82 g (R = 45%) de Z-Pro-Gly-OPFP:

$$[\alpha]_D^{20} = -36{,}8\ (c = 1\ dioxane)$$

On dissout ces 0,8 g dans 10 ml de dioxane et on ajoute simultanément 0,23 g (1,8 mmol) de chlorhydrate du glycinate d'éthyle et 0,25 g de triéthyle amine. On opère ensuite comme à l'exemple 55. On obtient 0,55 g (R = 94%) de Z-Pro-Gly-Gly-OEt.

$$F = 95 - 96°C\ [\alpha]_D^{20} = -21{,}4\ (C = 1\ EtOH)$$

que l'on recristallise dans un mélane acétate d'éthyle-hexane.

$$F = 117 - 118°C\ [\alpha]_D^{20} = -24{,}6\ (c = 1\ EtOH);$$

$$F_{Litt} = 120°C\ [\alpha]_D^{20} = -26\ (c = 1\ EtOH)$$

Exemple 58

Préparation du 2,2-diméthyle acrylate de N-succinimidyle

A 0,5 g d'acide 2,2-diméthylacrylique dans 15 ml de THF, on ajoute 1,63 g de carbonate de 1,2,2,2 tétrachloroéthyle et de N-succinimidyle, et 0,7 ml de triéthylamine. On agite 2 heures à 20°C et on lave avec une solution de chlorure de sodium. On sèche sur sulfate de magnésium et on évapore le solvant. On

obtient 0,6 g (R = 56%) de l'ester attendu RMN$^1$H ($CDCl_3$ TMS): 2,05 (s, $CH_3$); 2,2 (s, $CH_3$); 2,8 (s, $CH_2CH_2$); 5,9 (m, H−C = ).

Exemple 59

Préparation du 2-thiophène carboxylate de N-succinimidyle

On opère comme à l'exemple précédent. A partir de 0,64 g d'acide 2-thiophène carboxylique, on obtient 0,62 g (R = 51%) de l'ester attendu

F = 140 − 142°C

RMN$^1$H ($CDCl_3$, TMS): 2,8 ($CH_2CH_2$), 7,0 − 7,2 (m; H − C =); 7.6 (m; H − C =); 7.8 (m, H − C =)

**Revendications**

1. Procédé de préparation d'esters actifs caractérisé en ce qu'on fait réagir sur un acide carboxylique de formule Y—COOH dans laquelle Y est le reste d'un amino-acide N-protégé, d'un acide aliphatique en $C_1$ à $C_6$, saturé ou non, d'un acide hétérocyclique à 5 ou 6 chaînons, saturé ou non, les hétéroatomes étant choisis parmi le soufre ou l'azote, d'un acide aromatique hydrocarboné à 6 chaînons, en présence d'un agent de fixation d'acide chlorhydrique, un carbonate de formule

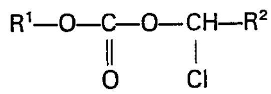

dans laquelle

$R^1$ représente un radical de formule

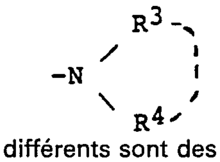

dans laquelle $R^3$ et $R^4$ identiques ou différents sont des radicaux hydrocarbonés pouvant comporter des atomes de carbone doublement liés à un atome d'oxygène et sont reliés entre eux pour former avec l'atome d'azote un hétérocycle azoté ayant jusqu'à 5 atomes de carbone dans le cycle, saturé ou non, qui peut être condensé avec un cycle phényle,

un radical benzotriazole, et

$R^2$ représente un radical aliphatique en $C_1$ à $C_5$ substitué ou non par un ou plusieurs atomes d'halogène ou le radical phényle substitué par un ou plusieurs atomes de chlore,

ou dans laquelle $R^1$ représente

un radical phényle substitué ou non par des atomes d'halogène et/ou des groupes nitro;

et $R^2$ représente un radical — $CCl_3$ ou un radical phényle substitué par un ou plusieurs atomes de chlore.

2. Procede selon la revendication précédente caractérisé en ce que $R^1$ est un groupe N-succinimidyle, 2,4,5 trichlorophényle, pentachloro ou pentafluorophényle, 4-nitrophényle, 2,4-dinitrophényle ou N-phtalimidyle.

3. Procédé selon l'une quelconque des revendications précédentes caractérisé en ce que l'agent de fixation d'acide chlorhydrique est une base organique ou minérale.

4. Procede selon la revendication précédente caractérisé en ce que la base est une amine tertiaire.

5. Procede selon la revendication précédente caractérisé en ce que l'amine tertiaire est la triéthylamine ou la N-méthylmorpholine.

6. Procédé selon l'une quelconque des revendications précédentes caractérisé en ce que les composés sont mis à réagir dans un solvant inerte choisi dans le groupe qui comprend les éthers cycliques ou non, les esters, les nitriles, les alcools, les amides et les cétones.

7. Procede selon la revendication précédente caractérisé en ce que le solvant est le tétrahydrofuranne.

8. Procédé selon l'une quelconque des revendications précédentes caractérisé en ce que la température de la réaction est comprise entre −20°C et 100°C.

9. Procede selon la revendication précédente caractérisé en ce que la température de la réaction est comprise entre 20 et 30°C.

10. Carbonates alpha-chlorés caractérisés en ce qu'ils ont pour formule

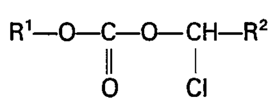

dans laquelle $R^1$ représente un radical de formule

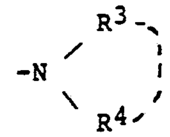

dans laquelle $R^3$ et $R^4$ identiques ou différents sont des radicaux hydrocarbonés pouvant comporter des atomes de carbone doublement liés à un atome d'oxygène et sont reliés entre eux pour former avec l'atome d'azote un hétérocycle azoté ayant jusqu'à 5 atomes de carbone dans le cycle, saturé ou non, qui peut être condensé avec un cycle phényle,

un radical benzotriazole, et

$R^2$ représente un radical aliphatique en $C_1$ à $C_5$ substitué ou non par un ou plusieurs atomes d'halogène ou le radical phényle substitué par un ou plusieurs atomes de chlore,

ou dans laquelle $R^1$ représente

un radical phényle substitué ou non par des atomes d'halogéne et/ou des groupes nitro;

et $R^2$ représente un radical — $CCl_3$ ou un rdical phényle substitué par un ou plusieurs atomes de chlore.

11. Carbonates selon la revendication précédente caractérisés en ce que $R^1$ est un groupe N-succinimidyle, 2,4,5-trichlorophényle, pentachloro ou pentafluorophényle, 4-nitrophényle, 2,4-dinitrophényle ou N-phtalimidyle.

12. Procédé de préparation des carbonates selon les revendications 10 ou 11 caractérisé en ce qu'on fait réagir un composé de formule $R^1OH$ dans laquelle $R^1$ a la signification précédente sur un chloroformiate alpha-chloré de formule

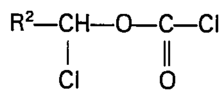

$R^2$ ayant les significations précédentes, dans un milieu solvant inerte à une température de −20° à 100°C en présence d'une base organique ou minérale.

13. Procédé selon la revendication précédente caractérisé en ce que la base est ajoutée au mélange des réactifs, progressivement.

14. Procédé selon la revendication 12 ou 13 caractérisé en ce que base est la pyridine ou la triéthylamine.

15. Procédé selon l'une quelconque des revendications 12 à 14 caractérisé en ce que la base est utilisée en quantité stoéchiométrique.

16. Procédé selon l'une quelconque des revendications 12 à 15 caractérisé en ce que le milieu solvant est constitué d'un ou de plusieurs solvants choisis parmi les solvants aliphatiques chlorés, les éthers cycliques ou non, les cétones, les nitriles, les esters et les hydrocarbures aliphatiques ou aromatiques.

17. Procédé selon l'une quelconque des revendications 12 à 16 caractérisé, en ce que lorsque $R^1$ est un phényle, le milieu solvant est un mélange benzène-éther de pétrole.

18. Procédé selon l'une quelconque des revendications 12 à 17 caractérisé en ce que la température de la réaction est comprise entre −10° et 40°C.

**Patentansprüche**

1. Verfahren zur Herstellung aktiver Ester, gekennzeichnet durch Umsetzung einer Carbonsäure der Formel Y-COOH, in der Y den Rest einer N-geschützten Aminosäure, einer gesättigten oder ungesättigten aliphatischen $C_{1-6}$-Carbonsäure, einer gesättigten oder ungesättigten fünf-oder sechsgliedrigen heterocyclischen Säure, deren Heteroatome unter Schwefel und Stickstoff ausgewählt sind, oder einer aromatischen Kohlenwasserstoffsäure mit sechs Ringatomen bedeutet,

mit einem Carbonat der Formel

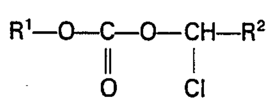

in der bedeuten:

$R^1$ eine Gruppe der Formel

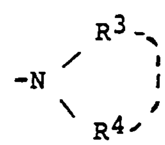

in der $R^3$ und $R^4$, die gleich oder verschieden sein können, Kohlenwasserstoffgruppen darstellen, die doppelt an ein Sauerstoffatom gebundene Kohlenstoffatome aufweisen können und so miteinander verbunden sind, daß sie mit dem Stickstoffatom einen gesättigten oder ungesättigten stickstoffhaltigen Heterocyclus mit bis zu fünf Kohlenstoffatomen im Ring bilden, der mit einem Phenylring kondensiert sein kann,

oder eine Benzotriazolgruppe und

$R^2$ eine ggfs. mit einem oder mehreren Halogenatomen substituierte aliphatische $C_{1-5}$-Gruppe oder mit einem oder mehreren Chloratomen substituiertes Phenyl, oder

$R^1$ eine ggfs. mit Halogenatomen und/oder Nitrogruppen substituierte Phenylgruppe und $R^2$ die Gruppe $-CCl_3$ oder eine mit einem oder mehreren Chloratomen substituierte Phenylgruppe,

in Gegenwart eines Mittels zur Bindung von Chlorwasserstoffsäure.

2. Verfahren nach Anspruch 1, dadurch gekennzeichnet, daß $R^1$ N-Succinimidyl, 2,4,5-Trichlorphenyl, Pentachlor- oder Pentafluorphenyl, 4-Nitrophenyl, 2,4-Dinitrophenyl oder N-Phthalimidyl bedeutet.

3. Verfahren nach Anspruch 1 oder 2, dadurch gekennzeichnet, daß das Mittel zur Bindung der Chlorwasserstoffsäure eine organische oder anorganische Base ist.

4. Verfahren nach Anspruch 3, dadurch gekennzeichnet, daß die Base ein tertiäres Amin ist.

5. Verfahren nach Anspruch 4, dadurch gekennzeichnet, daß das tertiäre Amin Triethylamin oder N-Methylmorpholin is.

6. Verfahren nach einem der vorhergehenden Ansprüche, dadurch gekennzeichnet, daß die Verbindungen in einem inerten Lösungsmittel umgesetzt werden, das unter offenkettigen und cyclischen Ethern, Estern, Nitrilen, Alkoholen, Amiden und Ketonen ausgewählt ist.

7. Verfahren nach Anspruch 6, dadurch gekennzeichnet, daß das Lösungsmittel Tetrahydrofuran ist.

8. Verfahren nach einem der vorhergehenden Ansprüche, dadurch gekennzeichnet, daß die Reaktionstemperatur im Bereich von −20° bis 100°C liegt.

9. Verfahren nach Anspruch 8, dadurch gekennzeichnet, daß die Reaktionstemperatur im Bereich von 20 bis 30°C liegt.

10. α-chlorierte Carbonate, gekennzeichnet durch die Formel

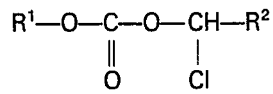

in der bedeuten:

$R^1$ eine Gruppe der Formel

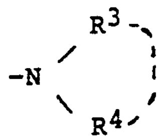

in der $R^3$ und $R^4$, die gleich oder verschieden sein können, Kohlenwasserstoffgruppen darstellen, die doppelt an ein Sauerstoffatom gebundene Kohlenstoffatome aufweisen können und so miteinander verbunden sind, daß sie mit dem Stickstoffatom einen gesättigten oder ungesättigten stickstoffhaltigen Heterocyclus mit bis zu fünf Kohlenstoffatomen im Ring bilden, der mit einem Phenylring kondensiert sein kann, oder

eine Benzotriazolgruppe und

$R^2$ eine ggfs. mit einem oder mehreren Halogenatomen substituierte aliphatische $C_{1-5}$-Gruppe oder mit einem oder mehreren Chloratomen substituiertes Phenyl, oder

$R^1$ eine ggfs. mit Halogenatomen und/oder Nitrogruppen substituierte Phenylgruppe und

$R^2$ die Gruppe $-CCl_3$ oder eine mit einem oder mehreren Chloratomen substituierte Phenylgruppe.

1. Carbonate nach Anspruch 10, dadurch gekennzeichnet, daß $R^1$ N-Succinimidyl, 2,4,5-Trichlorphenyl, Pentachlor- oder Pentafluorphenyl, 4-Nitrophenyl, 2,4-Dinitrophenyl oder N-Phthalimidyl ist.

12. Verfahren zur Herstellung der Carbonate nach Anspruch 10 oder 11, gekennzeichnet durch Umsetzung einer Verbindung der Formel $R^1OH$, in der $R^1$ die obige Bedeutung besitzt, mit einem α-chlorierten Chlorformiat der Formel

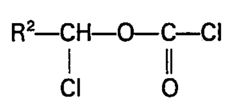

in der $R^2$ die oben angegebenen Bedeutungen besitzt, in einem inerten Lösungsmittelmedium bei einer Temperatur von −20 bis 100°C in Gegenwart einer organischen oder anorganischen Base.

13. Verfahren nach Anspruch 12, dadurch gekennzeichnet, daß die Base dem Reaktionsgemisch allmählich zugegeben wird.

14. Verfahren nach Anspruch 12 oder 13, dadurch gekennzeichnet, daß die Base Pyridin oder Triethylamin ist.

15. Verfahren nach einem der Ansprüche 12 bis 14, dadurch gekennzeichnet, daß die Base in stöchiometrischer Menge eingesetzt wird.

16. Verfahren nach einem der Ansprüche 12 bis 15, dadurch gekennzeichnet, daß das Lösungsmittelmedium aus einem oder mehreren Lösungsmitteln besteht, die unter aliphatischen chlorierten Lösungsmitteln, cyclischen oder offenkettigen Ethern, Ketonen, Nitrilen, Estern und aliphatischen oder aromatischen Kohlenwasserstoffen ausgewählt sind.

17. Verfahren nach einem der Ansprüche 12 bis 16, dadurch gekennzeichnet, daß, wenn $R^1$ Phenyl bedeutet, das Lösungsmittelmedium ein Benzol-Petrolether-Gemisch ist.

18. Verfahren nach einem der Ansprüche 12 bis 17, dadurch gekennzeichnet, daß die Reaktionstemperatur im Bereich von $-10$ bis 40°C liegt.

## Claims

1. Process for preparing active esters, characterized in that a carboxylic acid of formula Y—COOH, in which Y is the residue of an N-protected amino acid, of a saturated or unsaturated $C_1$ to $C_6$ aliphatic acid, of a saturated or unsaturated 5- or 6-ring-membered heterocyclic acid, the hetero atoms being chosen from sulphur or nitrogen, or of a 6-ring-membered hydrocarbon-based aromatic acid, is reacted, in the presence of an agent for binding hydrochloric acid, with a carbonate of formula

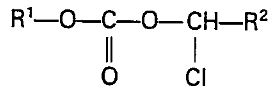

in which

$R^1$ denotes a radical of formula

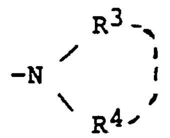

in which $R^3$ and $R^4$, which may be identical or different, are hydrocarbon radicals, which can contain carbon atoms doubly bonded to an oxygen atom and are joined together to form, with the nitrogen atom, a saturated or unsaturated nitrogenous heterocyclic system having up to 5 carbon atoms in the ring, which can be fused to a phenyl ring, a benzotriazole radical, and

$R^2$ denotes a $C_1$ to $C_5$ aliphatic radical which is unsubstituted or substituted with one or more halogen atoms or a phenyl radical substituted with one or more chlorine atoms, or in which $R^1$ denotes a phenyl radical which is unsubstituted or substituted with halogen atoms and/or nitro groups; and $R^2$ denotes a $—CCl_3$ radical or a phenyl radical substituted with one or more chlorine atoms.

2. Process according to the preceding claim, characterized in that $R^1$ is an N-succinimidyl, 2,4,5-trichlorophenyl, pentachloro- or pentafluorophenyl, 4-nitrophenyl, 2,4-dinitrophenyl or N-phthalimidyl group.

3. Process according to any one of the preceding claims, characterized in that the agent for binding hydrochloric acid is an organic or inorganic base.

4. Process according to the preceding claim, characterized in that the base is a tertiary amine.

5. Process according to the preceding claim, characterized in that the tertiary amine is triethylamine or N-methylmorpholine.

6. Process according to any one of the preceding claims, characterized in that the compounds are reacted in an inert solvent chosen from the group comprising ethers, cyclic or otherwise, esters, nitriles, alcohols, amides and ketones.

7. Process according to the preceding claim, characterized in that the solvent is tetrahydrofuran.

8. Process according to any one of the preceding claims, characterized in that the reaction temperature is between $-20$°C and 100°C.

9. Process according to the preceding claim, characterized in that the reaction temperature is between 20 and 30°C.

10. Alpha-chlorinated carbonates, characterized in that they are of the formula

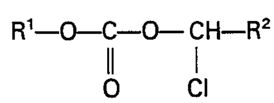

in which $R^1$ denotes a radical of formula

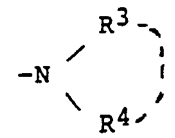

in which $R^3$ and $R^4$, which may be identical or different, are hydrocarbon radicals which can contain carbon atoms doubly bonded to an oxygen atom and are joined together to form, with the nitrogen atom, a saturated or unsaturated nitrogenous heterocyclic system having up to 5 carbon atoms in the ring, which can be fused to a phenyl ring, a benzotriazole radical, and

$R^2$ denotes a $C_1$ to $C_5$ aliphatic radical which is unsubstituted or substituted with one or more halogen atoms or a phenyl radical substituted with one or more chlorine atoms, or in which $R^1$ denotes a phenyl radical which is unsubstituted or substituted with halogen atoms and/or nitro groups; and

$R^2$ denotes a $—CCl_3$ radical or a phenyl radical substituted with one or more chlorine atoms.

11. Carbonates according to the preceding claim, characterized in that $R^1$ is an N-succinimidyl, 2,4,5-trichlorophenyl, pentachloro- or pentafluorophenyl, 4-nitrophenyl, 2,4-dinitrophenyl or N-phthalimidyl group.

12. Process for preparing the carbonates according to Claims 10 and 11, characterized in that a compound of formula $R^1OH$ in which $R^1$ has the meaning given above, is reacted with an alpha-chlorinated chloroformate of formula

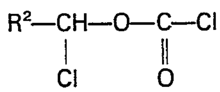

$R^2$ having the meanings given above, in an inert solvent medium at a temperature of −20° to 100°C, in the presence of an organic or inorganic base

13. Process according to the preceding claim, characterized in that the base is added gradually to the mixture of the reagents.

14. Process according to Claim 12 or 13, characterized in that the base is pyridine or triethylamine.

15. Process according to any one of Claims 12 to 14, characterized in that the base is used in a stoichiometric amount.

16. Process according to any one of Claims 12 to 15, characterized in that the solvent medium consists of one or more solvents chosen from chlorinated aliphatic solvents, ethers, cyclic or otherwise, ketones, nitriles, esters and aliphatic or aromatic hydrocarbons.

17. Process according to any one of Claims 12 to 16, characterized in that, when $R^1$ is a phenyl group, the solvent medium is a benzene/petroleum ether mixture.

18. Process according to any one of Claims 12 to 17, characterized in that the reaction temperature is between −10° and 40°C.